# FASCICULE DE BREVET EUROPEEN

(11) **EP 0 530 065 B1**
(45) Date de publication et mention de la délivrance du brevet: **13.09.1995**
(21) Numéro de dépôt: 92402152.0
(22) Date de dépôt: 24.07.1992
(51) Int. Cl.: A61K 7/043

(54) **Vernis à ongles contenant une amino-silicone**
Nagellack enthaltend eine Amino-Silicone
Nail lacquer containing an aminosilicone

(30) Priorité: 26.07.1991 FR 9109515
(43) Date de publication de la demande: 03.03.1993
(73) Titulaire: L'OREAL, 75008 Paris (FR)
(72) Inventeur: Mellul, Myriam, F-94240 L'Hay Les Roses (FR); de la Poterie, Valérie, F-94150 Rungis (FR)
(74) Mandataire: Stalla-Bourdillon, Bernard

(56) Documents cités:
- EP-A- 0 432 572
- FR-A- 2 379 280
- FR-A- 2 397 186

## Description

La présente invention a pour objet un vernis à ongles, incolore ou coloré, contenant en plus des ingrédients usuels, une amino-silicone.

Parmi les principales caractéristiques que doivent posséder les vernis à ongles, on doit tout particulièrement mentionner l'absence d'irritation de la peau et des ongles, l'obtention d'un film homogène et d'un bon brillant mais également une bonne adhérence sur la surface de l'ongle ainsi qu'une certaine flexibilité et résistance du film, ceci en vue d'éviter sa fragilité pouvant conduire à une craquelure du vernis.

De façon générale, on utilise à l'heure actuelle pour conférer une bonne adhérence, des résines modifiées et des plastifiants, ce qui conduit à une bonne flexibilité du vernis.

En vue d'améliorer l'adhérence, il a également été proposé l'application d'une précouche d'accrochage ou encore l'introduction de certains additifs permettant d'améliorer l'adhérence.

L'utilisation de silicone dans les vernis à ongles en tant qu'additif a déjà été décrite dans le but d'améliorer la résistance à l'eau et de faciliter l'étalement du vernis.

Ainsi, dans le brevet US 4.873.077, il a été proposé l'utilisation d'une silicone fluide en vue d'améliorer la douceur du film après évaporation du solvant et d'améliorer sa résistance à l'humidité.

De même, dans les demandes de brevet japonais n° 55 193316 et 59 199621, il a également été proposé l'utilisation de silicones, en particulier de méthyle ou diméthylpolysiloxane pour améliorer le brillant et la résistance à l'eau et à l'abrasion du film de vernis.

Dans le brevet Français (2.379.280) ont été décrites des compositions de vernis à ongles formant des films facilement détachables des ongles sans l'aide de produit dissolvant.

Selon ce brevet, la composition de vernis contient, en plus des ingrédients usuels, de 0,01 à 30 parties en poids par rapport à la résine naturelle ou synthétique d'un organopolysiloxane porteur de diverses fonctions dont éventuellement des fonctions amines. La présence de l'organopolysiloxane est essentielle en vue d'obtenir un film facilement détachable ou pelable de la surface de l'ongle.

Après d'importantes recherches, on a maintenant trouve, de façon tout à fait surprenante et inattendue, qu'en utilisant une certaine classe bien déterminée d'organopolysiloxanes à fonction(s) amine(s) ou amino-silicones dans une composition de vernis dont la substance filmogène est de la nitrocellulose et la résine est une résine aryl sulfonamide formaldéhyde ou une résine alkyde, il était possible d'améliorer de façon particulièrement significative, l'adhérence des vernis, incolores ou colorés, sur les ongles.

Donc, contrairement à l'enseignement du brevet Français 2.379.280 l'emploi d'une amino-silicone permet d'augmenter l'adhérence d'une composition de vernis, si toutefois la substance filmogène est de la nitrocellulose et la résine, une résine aryl sulfonamide formaldéhyde ou une résine alkyde.

La présente invention a donc pour objet à titre de produit industriel nouveau, un vernis à ongles, incolore ou coloré, contenant dans un système solvant pour vernis, de la nitrocellulose, une résine aryl sulfonamide formaldéhyde ou une résine alkyde et un agent plastifiant le dit vernis contenant en outre, en vue d'améliorer l'adhérence, de 0,05 à 5 % en poids d'une amino-silicone ayant la formule générale suivante : 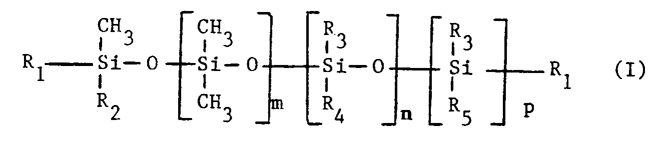
dans laquelle :
R₁ représente CH₃, OCH₃, OCH₂CH₃ ou (̵CH₂)̵_{d}NH₂,
R₂ représente CH₃, OCH₃, ou OCH₂CH₃,
R₃ représente CH₃, OCH₃, OCH₂CH₃ ou OSi (CH₃)₃,
R₄ représente CH₃ ou 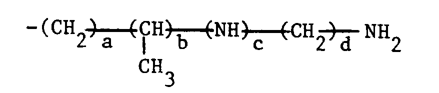
R₅ représente CH₃, OCH₃, OCH₂CH₃ ou 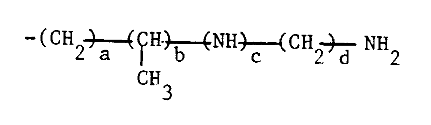
a est 0 à 6
b est 0 à 2
c est 0 ou 1
d est 1 à 6
sous réserve que :
(i) lorsque R₁ représente le radical -(̵CH₂)̵_{d}NH₂, R₄ représente CH₃ et R₅ représente CH₃, OCH₃ ou OCH₂CH₃,
(ii) lorsque R₄ représente CH₃, R₅ représente : 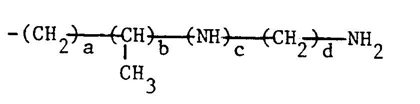 et,
(iii) lorsque R₄ représente 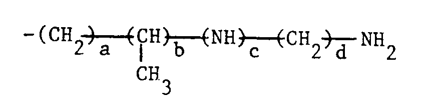 R₅ représente CH₃, OCH₃ ou OCH₂CH₃,
   m est 1 à 1000
   n est 0 à 50 et
   p est 0 à 50
   n et p ne pouvant simultanément être 0,
   le poids moléculaire de la dite amino-silicone étant compris entre 500 et 100.000 .

Selon l'invention, le pourcentage en amino-silicone de Formule (I) est de préférence compris entre 0,1 et 1,5 % en poids par rapport au poids total du vernis.

De préférence, l'amino-silicone doit présenter un indice d'amine (meq/g) compris entre 0,05 et 2,3 et de préférence entre 0,05 et 0,50.

Le poids moléculaire de l'amino-silicone de Formule (I) est de préférence compris entre 1000 et 20000.

Les études qui ont été réalisées ont permis par ailleurs de mettre en évidence que plus l'indice d'amine était faible, plus l'adhérence du vernis était améliorée.

Par ailleurs, il a été constaté que le poids moléculaire de l'amino-silicone joue un rôle important et que la tension superficielle, abaissée par la présence de la silicone, améliore l'étalement et l'adhérence.

Parmi les amino-silicones de Formule (I), on peut notamment citer celles vendues par la Société SHIN-ETSU sous les dénominations "KF860", "KF861", "KF862", "KF864", "KF865", "KF867" "X22-3680" et "X22-3801C", celles vendues par la Société WACKER sous les dénominations "SLM 55051", "L656", "SLM 55067", "VP1653" et "VP1480M" et celle vendue par la Société DOW CORNING sous la dénomination de "DC929", celles vendues par la Société GOLDSCHMIDT sous les dénominations de "TEGOMER A-Si2320" ou "TEGOMER A-Si2120", et celle vendue par la Société GENERAL ELECTRIC sous la dénomination de "SF 1706".

Les amino-silicones peuvent se présenter sous forme d'huile pure ou sous forme d'émulsion aqueuse comportant de 5 à 50 % de matière active tel que le produit "QS-7224" vendu par la Société DOW CORNING ou le produit "SLM 23047" vendu par la Société WACKER.

Selon l'invention, il est également possible d'associer l'amino-silicone telle que définie ci-dessus à une gomme de silicone ou une résine de silicone à faible pourcentage, de 0,1 à 2 % en poids et de préférence 0,3 % en poids pour améliorer l'étalement, l'aspect lisse du film et la brillance.

Selon l'invention, le système solvant du vernis est généralement présent en une proportion comprise entre 55 et 90 % en poids par rapport au poids total du vernis.

Ce système solvant est essentiellement constitué par un mélange de divers solvants organiques volatils, ceci en vue d'obtenir des temps de séchage relativement courts.

Parmi ces solvants on peut citer l'acétone, l'acétate d'éthyle, l'acétate de butyle, l'acétate de méthoxy-2 éthyle, la méthyl-éthyl-cétone, la méthyl-isobutyl-cétone, l'acétate de méthyle, l'acétate d'amyle et l'acétate d'isopropyle.

Le système solvant peut comprendre également un diluant qui est de préférence un hydrocarbure linéaire ou ramifié saturé, tel que l'hexane ou l'octane ou encore un hydrocarbure aromatique tel que le toluène ou le xylène dans une proportion généralement comprise entre 10 et 35 % par rapport au poids total du vernis.

Le système solvant peut également inclure d'autres solvants volatils tels que l'éthanol, du n-butanol, du n-propanol, de l'isopropanol ou leurs mélanges.

La nitrocellulose ou substance filmogène est généralement présente dans le vernis selon l'invention à une concentration comprise entre 5 et 20% et de préférence entre 10 et 20% en poids par rapport au poids total du vernis.

Parmi les nitrocelluloses particulièrement préférées, on doit citer celles du type "RS" ou "SS" et en particulier la nitrocellulose type 1/4 seconde RS, la nitrocellulose type 1/2 seconde RS, la nitrocellulose type 1/2 seconde SS et la nitrocellulose type 3/4 seconde RS.

Comme substance filmogène additionnelle on peut également utiliser selon l'invention des dérivés polyvinyliques tels que le butyrate de polyvinyle, ainsi que les copolymères décrits dans les brevets français n°80.07328, 81.03199 et 88.08172.

Selon l'invention, l'agent plastifiant est généralement présent dans le vernis à une concentration comprise entre 2 et 10% en poids par rapport au poids total du vernis.

Les agents plastifiants permettent de régler la flexibilité du film sans affaiblir sa résistance ou sa force physique. Parmi les agents plastifiants susceptibles d'être utilisés dans les vernis selon l'invention on peut citer : le phosphate de tricrésyle, le benzoate de benzyle, le phosphate de tributyle, l'acétyl-ricinoléate de butyle, l'acétyl-ricinoléate de glycéryle, le phtalate de dibutyle, le glycolate de butyle, le phtalate de dioctyle, le stéarate de butyle, le phosphate de tributoxy- éthyle, le phosphate de triphényle, le citrate de triéthyle, le citrate de tributyle, l'acétyl-citrate de tributyle, l'acétyl-citrate de triéthyl-2 hexyle, le tartrate de dibutyle, le phtalate de diméthoxyéthyle, le phtalate de di-isobutyle, le phtalate de diamyle, le camphre, le triacétate de glycérol et leurs mélanges.

Selon l'invention, la résine aryl sulfonamide formaldéhyde ou la résine alkyde est généralement présente en une proportion comprise entre 0,5 et 15 % en poids par rapport au poids total du vernis.

Parmi les résines du type aryl-sulfonamide formaldéhyde on peut notamment citer la résine toluène sulfonamide formaldéhyde plus connue sous les dénominations commerciales de "Santolite MHP", "Santolite MS 80%" et "Ketjenflex MS80" et parmi les résines alykydes celles vendues par la Société DAINIPPON sous la dénomination de "BECKOSOL ODE 230-70".

Ces résines tout en augmentant le pouvoir filmogène améliorent le brillant ainsi que l'adhérence.

Lorsque le vernis à ongles selon l'invention est un vernis coloré, on utilise alors au moins un pigment de nature organique ou inorganique.

Parmi les pigments organiques, on peut citer les D & C Red n°10, 11, 12 et 13, le D & C Red n°7 les D & C Red n° 5 et 6, les D & C Red n°34, des laques telles que la laque D & C Yellow n°5 et la laque D & C Red n°2.

Parmi les pigments inorganiques, on peut citer le dioxyde de titane, l'oxychlorure de bismuth, l'oxyde de fer brun et les les oxydes de fer rouge. On peut citer comme pigment organique la guanine.

Selon cette forme de réalisation, les pigments sont généralement présents en une proportion comprise entre 0,01 et 2% en poids par rapport au poids total du vernis.

Enfin, en vue d'éviter la sédimentation des pigments, certains agents thixotropes peuvent être employés, tels que par exemple la "Bentone 27" ou la "Bentone 38".

Les vernis à ongles selon l'invention peuvent en outre contenir des adjuvants couramment utilisés dans les vernis à ongles. Parmi ces adjuvants, on peut citer les filtres U.V. tels que les dérivés de benzophénone et le 2-cyano-3,3-diphényl acrylate d'éthyle.

On va maintenant donner à titre d'illustration et sans aucun caractère limitatif plusieurs exemples de vernis à ongles selon l'invention.

### EXEMPLE 1 : Vernis à ongles coloré

| | |
|---|---|
| - Nitrocellulose | 10,82 |
| - Résine toluène sulfonamide formaldéhyde ("Ketjenflex MS80" vendu par AKZO) | 9,74 |
| - Acétylcitrate de tributyle ("Citroflex A4" vendu par PFIZER) | 6,495 |
| - Toluène | 30,91 |
| - Acétate de butyle | 21,64 |
| - Acétate d'éthyle | 9,27 |
| - Alcool isopropylique | 7,72 |
| - Stéaralkonium hectorite | 1,35 |
| - Pigments | 1,00 |
| - Amino-silicone ("KF865" vendu par SHIN-ETSU) | 1,00 |
| - Acide citrique | 0,055 |

La présence dans ce vernis de l'amino-silicone permet d'améliorer l'adhérence de + 39,4 % par rapport au même vernis ne contenant pas d' amino-silicone.

### EXEMPLE 2 : Vernis à ongles coloré

| | |
|---|---|
| - Nitrocellulose | 10,9 |
| - Résine toluène sulfonamide formaldéhyde ("Ketjenflex MS80" vendu par AKZO) | 9,8 |
| - Acétylcitrate de tributyle ("Citroflex A4" vendu par PFIZER) | 6,495 |
| - Toluène | 31,105 |
| - Acétate de butyle | 21,8 |
| - Acétate d'éthyle | 9,395 |
| - Alcool isopropylique | 7,8 |
| - Stéaralkonium hectorite | 1,35 |
| - Pigments | 1,00 |
| - Amino-Silicone ("KF865" vendu par SHIN-ETSU) | 0,3 |
| - Acide citrique | 0,055 |

Amélioration de l'adhérence : + 33,18 %

### EXEMPLE 3 : Vernis à ongles coloré

| | |
|---|---|
| - Nitrocellulose | 9,8 |
| - Résine alkyde | 10,5 |
| - Acétylcitrate de tributyle ("Citroflex A4" vendu par PFIZER) | 7,7 |
| - Xylène | 4,5 |
| - Toluène | 28,05 |
| - Acétate de butyle | 19,625 |
| - Acétate d'éthyle | 8,42 |
| - Alcool isopropylique | 8,00 |
| - Stéaralkonium hectorite | 1,35 |
| - Pigments | 1,00 |
| - Amino-silicone ("KF865" vendu par SHIN-ETSU) | 1,00 |
| - Acide citrique | 0,055 |

Amélioration de l'adhérence : + 73,3%

### EXEMPLE 4 : Vernis à ongles incolore

| | |
|---|---|
| - Nitrocellulose | 12,00 |
| - Résine toluène sulfonamide-formaldéhyde ("Ketjenflex MS80" vendue par le AKZO) | 9,00 |
| - Camphre | 1,00 |
| - Phtalate de dibutyle | 6,05 |
| - Toluène | 29,38 |
| - Acétate de butyle | 24,00 |
| - Acétate d'éthyle | 9,05 |
| - Alcool isopropylique | 6,00 |
| - Stéaralkonium hectorite | 1,00 |
| - Amino-silicone ("KF865" vendu par SHIN ETSU) | 1,50 |
| - Acide citrique | 0,02 |

Amélioration de l'adhérence : + 41,4%

### EXEMPLE 5 : Vernis à ongles coloré

| | |
|---|---|
| - Nitrocellulose | 10,85 |
| - Résine toluène sulfonamide formaldéhyde ("Ketjenflex MS80" vendu par AKZO") | 9,75 |
| - Acétyltributyle citrate ("Citroflex A4 vendu par PFIZER) | 6,46 |
| - Toluène | 30,94 |
| - Acétate de butyle | 21,685 |
| - Acétate d'éthyle | 9,35 |
| - Alcool isopropylique | 7,76 |
| - Stéaralkonium hectorite | 1,35 |
| - Pigments | 1,00 |
| - Amino-silicone ("KF865" vendu par SHIN ETSU) | 0,30 |
| - Gomme de silicone ("TP232" vendue par UNION CARBIDE) | 0,50 |
| - Acide citrique | 0,055 |

Amélioration de l'adhérence : + 35,9 %

### EXEMPLE 6 : Vernis à ongles coloré

| | |
|---|---|
| - Nitrocellulose | 10,87 |
| - Résine toluène sulfonamide formaldéhyde ("Ketjenflex MS80" vendu par AZKO | 9,77 |
| - Acétyltributyle citrate ("Citroflex A4" vendu par PFIZER) | 6,47 |
| - Toluène | 31,00 |
| - Acétate de butyle | 21,735 |
| - Acétate d'éthyle | 9,37 |
| - Alcool isopropylique | 7,78 |
| - Stéaralkonium hectorite | 1,35 |
| - Pigments | 1,00 |
| - Amino-silicone en émulsion ("SLM 23047" vendu par WACKER) | 0,60 |
| - Acide citrique | 0,055 |

Amélioration de l'adhérence : + 36,9

### EXEMPLE 7 : Vernis à ongles coloré

| | |
|---|---|
| - Nitrocellulose | 10,90 |
| - Résine toluène sulfonamide formaldéhyde ("Ketjenflex MS80" vendu par AZKO | 9,80 |
| - Acétyltributyle citrate ("Citroflex A4" vendu par PFIZER) | 6,495 |
| - Toluène | 31,105 |
| - Acétate de butyle | 21,80 |
| - Acétate d'éthyle | 9,395 |
| - Alcool isopropylique | 7,80 |
| - Stéaralkonium hectorite | 1,35 |
| - Pigments | 1,00 |
| - Amino-silicone ("X22-161C" vendu par SHIN-ETSU) | 0,30 |
| - Acide citrique | 0,055 |

Amélioration de l'adhérence : + 22,4 %

### EXEMPLE 8 : Vernis à ongles coloré

| | |
|---|---|
| - Nitrocellulose | 9,8 |
| - Résine alkyde ("BECKOSOL ODE-230-70" vendu par DAINIPPON | 10,5 |
| - Acétylcitrate de tributyle ("Citroflex A4" vendu par PFIZER) | 7,7 |
| - Xylène | 4,5 |
| - Toluène | 28,05 |
| - Acétate de butyle | 19,625 |
| - Acétate d'éthyle | 8,42 |
| - Alcool isopropylique | 8,00 |
| - Stéaralkonium hectorite | 1,35 |
| - Pigments | 1,00 |
| - Amino-silicone ("KF865" vendu par SHIN-ETSU) | 0,50 |
| - Acide citrique | 0,055 |

Amélioration de l'adhérence : + 42,3%

## Revendications

1. Vernis à ongles, incolore ou coloré, contenant dans un système solvant pour vernis, de la nitrocellulose, une résine aryl sulfonamide formaldéhyde ou une résine alkyde et un agent plastifiant le dit vernis contenant en outre, en vue d'améliorer l'adhérence, de 0,05 à 5 % en poids d'une amino-silicone ayant la formule générale suivante : 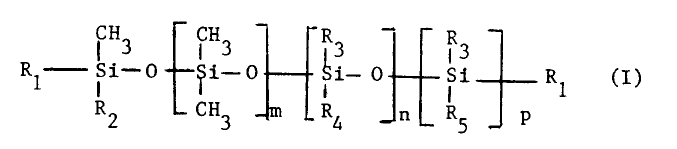 dans laquelle :
R₁ représente CH₃, OCH₃, OCH₂CH₃ ou (̵CH₂)̵_{d} NH₂,
R₂ représente CH₃, OCH₃, ou OCH₂CH₃,
R₃ représente CH₃, OCH₃, OCH₂CH₃ ou OSi (CH₃)₃,
R₄ représente CH₃ ou 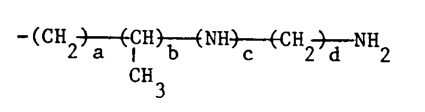 R₅ représente CH₃, OCH₃, OCH₂CH₃ ou 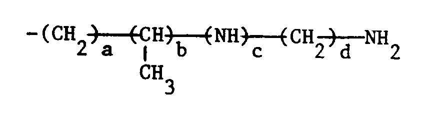 a est 0 à 6
b est 0 à 2
c est 0 ou 1
d est 1 à 6
sous réserve que :
(i) lorsque R₁ représente le radical _(CH₂)̵_{d}NH₂, R₄ représente CH₃ et R₅ représente CH₃, OCH₃ ou OCH₂CH₃,
(ii) lorsque R₄ représente CH₃, R₅ représente : 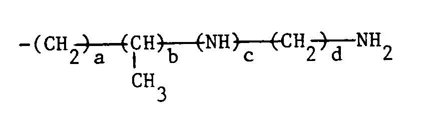 et,
(iii) lorsque R₄ représente 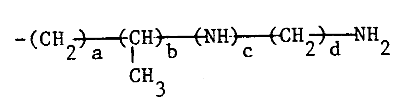 R₅ représente CH₃, OCH₃ ou OCH₂CH₃,
m est 1 à 1000
n est 0 à 50 et
p est 0 à 50
n et p ne pouvant simultanément être 0,
le poids moléculaire de la dite amino-silicone étant compris entre 500 et 100.000.

2. Vernis à ongles selon la revendication 1, caractérisé par le fait qu'il contient de préférence entre 0,1 et 1,5 % en poids d'amino-silicone de Formule (I) par rapport au poids total du vernis.

3. Vernis à ongles selon l'une quelconque des revendications 1 et 2, caractérisé par le fait que l'amino-silicone de formule (I) présente un indice d'amine (meq/g) compris entre 0,05 et 2,3 et un poids moléculaire compris de préférence entre 1.000 et 20.000.

4. Vernis à ongles selon la revendication 1, caractérisé par le fait que le système solvant est présent en une proportion comprise entre 55 et 90 % en poids par rapport au poids total du vernis.

5. Vernis à ongles selon la revendication 1, caractérisé par le fait que la nitrocellulose est présente à une concentration comprise entre 5 et 20 % en poids par rapport au poids total du vernis.

6. Vernis à ongles selon la revendication 1 caractérisé par le fait que la résine du type aryl sulfonamide formaldéhyde ou la résine alkyde est présente en une proportion comprise entre 0,5 et 15 % en poids par rapport au poids total du vernis.

7. Vernis à ongles selon l'une quelconque des revendications 1 à 6, caractérisé par le fait que la résine aryl sulfonamide formaldéhyde est la résine toluène sulfonamide formaldéhyde.

8. Vernis à ongles selon la revendication 1, caractérisé par le fait que l'agent plastifiant est présent à une concentration comprise entre 2 et 10 % en poids par rapport au poids total du vernis.

9. Vernis à ongles selon l'une quelconque des revendications précédentes, caractérisé par le fait qu'il contient au moins un pigment de nature organique ou inorganique.

10. Vernis à ongles selon la revendication 9, caractérisé par le fait que le pigment est présent à une concentration comprise entre 0,01 et 2 % par rapport au poids total du vernis.

## Claims

1. Coloured or colourless nail varnish containing, in a solvent system for varnishes, nitrocellulose, an arylsulphonamide formaldehyde resin or an alkyd resin and a plasticizer, the said varnish additionally containing, for the purpose of improving the adhesion, from 0.05 to 5% by weight of an amino-silicone having the following general formula: 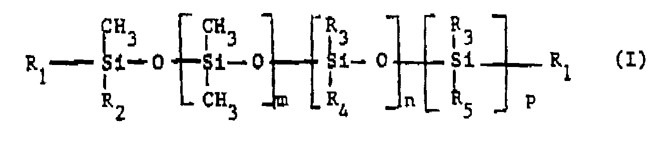 in which:
R₁ represents CH₃, OCH₃, OCH₂CH₃ or (̵CH₂)̵_{d}NH₂,
R₂ represents CH₃, OCH₃ or OCH₂CH₃,
R₃ represents CH₃, OCH₃, OCH₂CH₃ or OSi(CH₃)₃,
R₄ represents CH₃ or 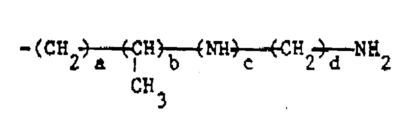 R₅ represents CH₃, OCH₃, OCH₂CH₃ or 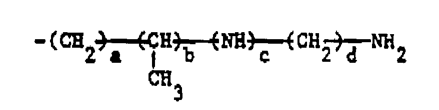 a is 0 to 6
b is 0 to 2
c is 0 or 1
d is 1 to 6
with the proviso that:
(i) when R₁ represents the radical -(CH₂)̵_{d}NH₂, R₄ represents CH₃ and R₅ represents CH₃, OCH₃ or OCH₂CH₃,
(ii) when R₄ represents CH₃, R₅ represents: 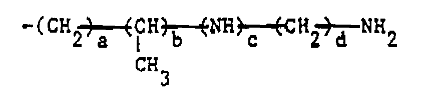 and,
(iii) when R₄ represents 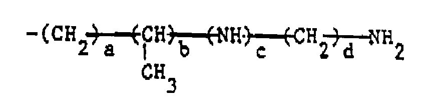 R₅ represents CH₃, OCH₃ or OCH₂CH₃,
m is 1 to 1000
n is 0 to 50 and
p is 0 to 50
it not being possible for n and p simultaneously to be 0,
the molecular weight of the said amino-silicone being between 500 and 100,000.

2. Nail varnish according to Claim 1, characterized in that it preferably contains between 0.1 and 1.5% by weight of amino-silicone of formula (I) relative to the total weight of the varnish.

3. Nail varnish according to either of Claims 1 and 2, characterized in that the amino-silicone of formula (I) has an amino number (meq/g) between 0.05 and 2.3 and a molecular weight preferably between 1000 and 20,000.

4. Nail varnish according to Claim 1, characterized in that the solvent system is present in a proportion between 55 and 90% by weight relative to the total weight of the varnish.

5. Nail varnish according to Claim 1, characterized in that the nitrocellulose is present at a concentration between 5 and 20% by weight relative to the total weight of the varnish.

6. Nail varnish according to Claim 1, characterized in that the resin of the arylsulphonamide formaldehyde type or the alkyd resin is present in a proportion between 0.5 and 15% by weight relative to the total weight of the varnish.

7. Nail varnish according to any one of Claims 1 to 6, characterized in that the arylsulphonamide formaldehyde resin is the toluenesulphonamide formaldehyde resin.

8. Nail varnish according to Claim 1, characterized in that the plasticizer is present at a concentration between 2 and 10% by weight relative to the total weight of the varnish.

9. Nail varnish according to any one of the preceding claims, characterized in that it contains at least one pigment of organic or inorganic nature.

10. Nail varnish according to Claim 9, characterized in that the pigment is present at a concentration between 0.01 and 2% relative to the total weight of the varnish.

## Patentansprüche

1. Farbloser der gefärbter Nagellack, der in einem Lösungsmittelsystem für Nagellacke Nitrocellulose, ein Arylsulfonamid-Formaldehyd-Harz oder ein Alkydharz sowie einen Weichmacher enthält, wobei der Nagellack zusätzlich zur Haftungsverbesserung 0,05 bis 5 Gew.-% eines Aminosilikons der allgemeinen Formel enthält: 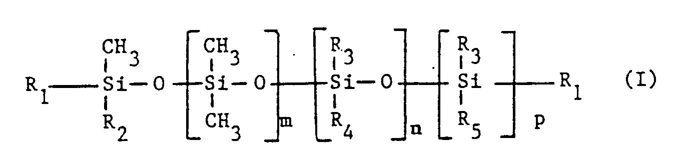 in der
R₁ CH₃, OCH₃, OCH₂CH₃ oder -(CH₂)_{d}-NH₂,
R₂ CH₃, OCH₃ oder OCH₂CH₃,
R₃ CH₃, OCH₃, OCH₂CH₃ oder OSi(CH₃)₃,
R₄ CH₃ oder 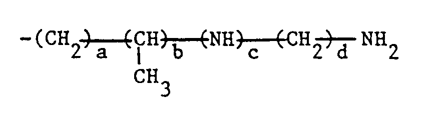 und
R₅ CH₃, OCH₃, OCH₂CH₃ oder 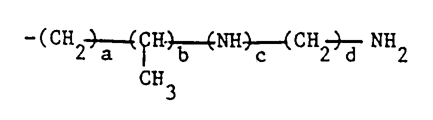 darstellen, wobei
a 0 bis 6, b 0 bis 2, c 0 oder 1 und d 1 bis 6 bedeutet,
mit der Maßgabe, daß
(i), wenn R₁ den Rest -(CH₂)_{d}-NH₂ darstellt, R₄ CH₃ und R₅ CH₃, OCH₃ oder OCH₂CH₃ sind,
(ii), wenn R₄ CH₃ ist, R₅ den Rest 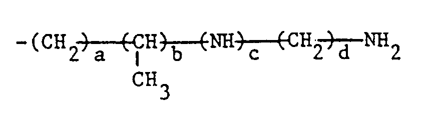 darstellt und
(iii) dann, wenn R₄ den Rest 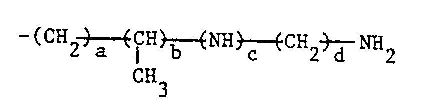 darstellt, R₅ CH₃, OCH₃ oder OCH₂CH₃ ist,
und
m 1 bis 1000, n 0 bis 50 und p 0 bis 50 ist, wobei n und p nicht gleichzeitig gleich 0 sein können und
wobei das das Molekulargewicht des Aminosilikons zwischen 500 und 100 000 liegt.

2. Nagellack gemäß Anspruch 1, dadurch gekennzeichnet, daß er, bezogen auf das Gesamtgewicht des Lackes, vorzugsweise zwischen 0,1 und 1,5 Gew.-% des Aminosilikons der Formel (I) enthält.

3. Nagellack gemäß einem der Ansprüche 1 und 2, dadurch gekennzeichnet, daß das Aminosilikon der Formel (I) einen Aminindex (mÄq/g) zwischen 0,05 und 2,3 sowie ein Molekulargewicht vorzugsweise von 1 000 bis 20 000 aufweist.

4. Nagellack gemäß Anspruch 1, dadurch gekennzeichnet, das das Lösungsmittelsystem einen Anteil von 55 bis 90 Gew.-%, bezogen auf das Gesamtgewicht des Lackes, ausmacht.

5. Nagellack gemäß Anspruch 1, dadurch gekennzeichnet, das die Nitrocellulose in einer Konzentration von 5 bis 20 Gew.-%, bezogen auf das Gesamtgewicht des Lackes, vorliegt.

6. Nagellack gemäß Anspruch 1, dadurch gekennzeichnet, daß das Harz vom Typ der Arylsulfon-Formaldehyd-Harze oder das Alkydharz in einer Menge von 0,5 bis 15 Gew.-%, bezogen auf das Gesamtgewicht des Lackes, vorliegt.

7. Nagellack gemäß einem der Ansprüche 1 bis 6, dadurch gekennzeichnet, daß das Arylsulfonamid-Formaldehyd-Harz ein Toluolsulfonamid-Formaldehyd-Harz ist.

8. Nagellack gemäß Anspruch 1, dadurch gekennzeichnet, daß der Weichmacher in einer Konzentration Von 2 bis 10 Gew.-%, bezogen auf das Gesamtgewicht des Lackes, vorliegt.

9. Nagellack gemäß einem der vorstehenden Ansprüche, dadurch gekennzeichnet, daß er mindestens ein organisches oder anorganisches Pigment enthält.

10. Nagellack gemäß Anspruch 9, dadurch gekennzeichnet, daß das Pigment in einer Konzentration von 0,01 bis 2 Gew.-%, bezogen auf das Gesamtgewicht des Lackes, vorliegt.
